# EUROPÄISCHE PATENTSCHRIFT

(11) **EP 0 638 538 B1**
(45) Veröffentlichungstag und Bekanntmachung des Hinweises auf die Patenterteilung: **28.10.1998**
(21) Anmeldenummer: 94110715.3
(22) Anmeldetag: 09.07.1994
(51) Int. Cl.: C07C 53/08, C07C 51/487, C07C 51/44

(54) **Verfahren zur Entfernung von Aldehyden und Acetalen aus technisch hergestellter Essigsäure**
Process for removing of aldehydes and acetals from technical-grade acetic acid
Procédé d'élimination des aldéhydes et des acétals à partir d'acide acétique du grade technique

(30) Priorität: 12.08.1993 DE 4327011
(43) Veröffentlichungstag der Anmeldung: 15.02.1995
(73) Patentinhaber: Celanese GmbH, 60439 Frankfurt (DE)
(72) Erfinder: Seidel, Andreas, Dr., D-50939 Köln (DE); Hauser, Alfred, Dr., D-50374 Erftstadt (DE); Prinz, Peter, D-50354 Hürth (DE)

(56) Entgegenhaltungen:
- DE-C- 307 550
- DE-C- 892 894
- US-A- 2 273 459
- US-A- 2 651 604
- US-A- 2 703 309
- US-A- 3 196 176
- US-A- 4 230 887

## Beschreibung

Die vorliegende Erfindung betrifft ein Verfahren zur Entfernung von Aldehyden und Acetalen aus technisch hergestellter Essigsäure, wie sie insbesondere bei der katalytischen Oxidation von Acetaldehyd und der Carbonylierung von Methanol anfällt.

Technisch hergestellte Essigsäure enthält je nach Produktionsverfahren Verunreinigungen wie Ameisensäure, Essigsäureester, Ameisensäureester, Acetaldehyd und Formaldehyd. Zur Erlangung höherer Spezifikationsstufen wie etwa der Qualität "chemisch rein" muß der Gehalt dieser Verunreinigungen in der Essigsäure weitestgehend reduziert werden. Ein analytisches Maß hierfür ist der Chromsäuretest nach dem im Deutschen Arzneibuch lo (1991) festgelegten Analysen-Verfahren, mit welchem der Gehalt oxidierbarer Verunreinigungen in der Essigsäure quantitativ bestimmt wird.

Neben den genannten destillativ abtrennbaren Verbindungen gibt es auch Verunreinigungen, die im Bereich des Siedepunkts der Essigsäure sieden und daher destillativ nicht entfernt werden können. Hierbei handelt es sich vor allem um die Additions- oder Kondensationsprodukte von Aldehyden, insbesondere oxidierbare Halb- und Vollacetale wie beispielsweise das Formaldehydmethylacetylacetal (Essigsäuremethoxymethylester; Kp. 117-118 °C) oder Acetaldehyddiacetylacetal (1.1-Diacetoxyethan; Kp. 169 °C). Da auch diese Verbindungen durch den Chromsäuretest miterfaßt werden, sind mittels einer Destillation nur unbefriedigende Ausbeuten an spezifikationsgerechter chemisch reiner Essigsäure erhältlich.

Die US-A-2,273,459 beschreibt ein Verfahren zur Reinigung von Essigsäure aus natürlichen Polyoxyalkylenglycole oder deren Ether Materialien durch Zugabe von bestimmten Säuren und anschließender Destillation des so erhaltenen Produktes. Hiermit wird offenbar die Aufgabe gelöst, natürliche Unreinheiten aus natürlicher Essigsäure sowie Verfärbungen aus dieser zu entfernen.

Die DE-C-307 550 beschreibt grundsätzlich ein Verfahren zur Reinigung von Essigsäure, bei dem insbesondere Ameisensäure durch Einsatz größerer Mengen an Schwefelsäure aus der vorgenannten Essigsäure entfernt wird.

Die US-A-4,230,887 beschreibt ein Verfahren zur Entfernung von Essigsäure aus wasserhaltigen Reaktionsgasgemischen, etwa bei solchen, bei denen die Essigsäureherstellung durch Oxidation von Alkanen, wie Butan, erfolgt ist. Als Entfernungssubstanzen werden verwendet.

Zur Entfernung der Aldehyde und Acetale wird deshalb in der US-A-4 061 546 Kaliumdichromat und in der DE-A-21 04 828 Kaliumpermanganat als Oxidationsmittel vorgeschlagen. In einer nachgeschalteten Destillation wird dann reine Essigsäure erhalten.

Nachteilig bei diesen Verfahren ist der Einsatz teurer Oxidationsmittel, die zu schwermetallhaltigen Destillationsrückständen führen, deren Entsorgung problematisch ist. Zudem können in derartigen Oxidationsprozessen auch toxische Produkte, beispielsweise Ameisensäure aus Formaldehyd, entstehen.

Es war daher die Aufgabe gestellt, ein kostengünstiges Verfahren zu finden, das eine möglichst vollständige Entfernung von Aldehyden und Acetalen aus technisch hergestellter Essigsäure ermöglicht, bei dem es keine Entsorgungsprobleme gibt und keine toxischen Produkte gebildet werden.

Überraschend konnte die Aufgabe erfindungsgemäß dadurch gelöst werden, daß man die technisch hergestellte Essigsäure in Gegenwart von einer Brönstedt-Säure wie Schwefelsäure, Methansulfonsäure, Toluolsulfonsäure oder Phosphorsäure in einer Menge von 0,005 bis 0,05 mol/l verunreinigter Essigsäure von 0,05 bis 1 Gewichtsprozent Wasser sowie von 3 bis 10 Äquivalenten, berechnet auf Aldehyd und Acetal, eines hochsiedenden Polyols mit dem allgemeinen Strukturglied 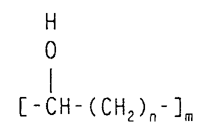 worin n für eine ganze Zahl von 0 bis 3 und m für eine ganze Zahl von größer als 3 steht, bei Temperaturen von 50 bis 118 °C über einen Zeitraum von 1 bis 5 Stunden reagieren läßt, und danach die reine Essigsäure durch fraktionierte Destillation aus dem Reaktionsprodukt gewinnt.

Das Verfahren der Erfindung kann weiterhin auch dadurch gekennzeichnet sein, daß man als hochsiedendes Polyol Polyvinylalkohol, Rohrzukker oder Dextrose, Stärke oder Zellulose einsetzt.

Vorteilhaft wird die technisch hergestellte Essigsäure mit einem Überschuß des hochsiedenden Polyols umgesetzt, um eine möglichst vollständige Entfernung der Aldehyde und Acetale zu gewährleisten.

Polyol, welches durch Verseifung von Polyvinylacetat in Methanol erhalten wird, kann vorteilhaft eingesetzt werden.

Das Beispiel 1 und die Vergleichsbeispiele 1,2 wurden mit einer technisch hergestellten Essigsäure durchgeführt, welche folgende Verunreinigungen enthielt:

| Essigsäuregehalt | 99,6 [Gew%] |
|---|---|
| Wasser | 0,036 [Gew%] |
| Formaldehyd | 57 [ppm] |
| Acetaldehyd | 1,8 [ppm] |
| Methylacetat | 59 [ppm] |
| Ethylacetat | 151 [ppm] |
| i-Propylacetat | 40 [ppm] |
| Essigsäuremethoxymethylester | 130 [ppm] |
| Chromsäuretest, nach Schering (%kg steht für "pro 1oo kg") | 80 [g CrO₃ %kg] |

Die Erfindung wird durch die Beispiele näher erläutert.

### Vergleichsbeispiel 1 (ohne Polyol-Zusatz)

5 kg technisch hergestellte Essigsäure wurden zusammen mit 3,5 g Wasser und 5 g Methansulfonsäure in den Verdampfer einer Dampfprallbodenkolonne mit 2o Böden gegeben und bei einem Kopfdruck von 35o mbar 3 h unter Rückfluß erhitzt. Danach wurde bei einem Kopfdruck von 35o mbar mit gegen Ende abnehmendem Rücklaufverhältnis (1o:1 bis 2:1) fraktionierend destilliert. Durch Vereinigung einzelner Fraktionen erhielt man insgesamt nach einem Leichtsiedervorlauf von o,54 kg (inclusive Kühlfallenprodukt) 1,68 kg Essigsäure (33,6 Gew% vom Einsatz) mit einem Chromsäuretest von 21 g CrO₃ %kg und 2,73 kg Essigsäure (54,6 Gew% vom Einsatz) mit einem Chromsäuretest < 1o g CrO₃ %kg.

### Beispiel 1 (mit Polyol-Zusatz)

5 kg technisch hergestellte Essigsäure wurden zusammen mit 3,5 g Wasser, 5 g Methansulfonsäure und o,9 g MOWIOL^{@}lo-74 (10fach valenter Überschuß berechnet auf Aldehyd und Acetal) in einem 6 l-Glaskolben mit einer aufgesetzten 20 bödigen Dampfprallbodenkolonne bei einem Kopfdruck von 35o mbar 3 Stunden bei einer Sumpftemperatur von 87,5 °C unter Rückfluß erhitzt. Die fraktionierte Destillation bei einem Rücklaufverhältnis von 1o:1 ergab 35o g eines wasserhaltigen Vorlaufs mit einem Chromsäuretest von 216 g CrO₃ %kg. Anschließend erhielt man bei Sumpftemperaturen zwischen 9o °C und 112 °C (89 °C bis 9o °C auf Boden 1o der Kolonne, Kopftemperatur 87 °C) mit abnehmendem Rücklaufverhältnis (1o:1 bis 2:1) 452o g Essigsäure mit einem Chromsäuretest von 7 g CrO₃ %kg (DAB lo-Qualität: < 32 g CrO₃ %kg) und der in der Tabelle 1 aufgeführten Analyse. Dies sind 9o % der eingesetzten Essigsäure. Weitere 23 g finden sich in der Destillationskühlfalle, der Rest verbleibt als Destillationssumpf.

**TABELLE 1**

| | gereinigte Essigsäure |
|---|---|
| Essigsäuregehalt | 99,8 [%] |
| Wasser | 0,18 [%] |
| Formaldehyd | < 10 [ppm] |
| Acetaldehyd | 0,2 [ppm] |
| Methylacetat | < 10 [ppm] |
| Ethylacetat | < 10 [ppm] |
| i-Propylacetat | < 10 [ppm] |
| Essigsäuremethoxymethylester | < 10 [ppm] |
| Chromsäuretest nach Schering | 7 [g CrO₃ %kg] |

### Vergleichsbeispiel 2 (Nur Destillationsreinigung)

In den Verdampfer einer Kolonne mit 3o Böden wurden 5oo g technisch hergestellte Essigsäure gegeben. Unter vollständigem Rückfluß wurde der Verdampferinhalt bei einem Kolonnenkopfdruck von 23o mbar auf 85 °C erhitzt. Sobald die Kolonne im Gleichgewicht war, wurde bei einem Rückfluß von 6:1 fraktionierend destilliert. Nach Abnahme eines Vorlaufs von 83 g erhielt man eine Fraktion von 167 g mit einem Chromsäuretest von < 32 g CrO₃ %kg. Die folgenden 139 g Destillat hatten mit einem Chromsäuretest von < 1o g CrO₃ %kg eine noch verbesserte Reinheit. Im Anschluß an diese Fraktion erhielt man weitere 83 g mit einem Chromsäuretest von < 32 g CrO₃ %kg. 27 g verblieben als Sumpfprodukt. Insgesamt wurden 5o,o % der eingesetzten Essigsäure mit einem Chromsäuretest < 32 g CrO₃ %kg und 28,o % der eingesetzten Essigsäure mit einem Chromsäuretest < 1o g CrO₃ %kg erhalten.

## Patentansprüche

1. Verfahren zur Entfernung von Aldehyden und Acetalen aus technisch hergestellter Essigsäure, dadurch gekennzeichnet, daß man die verunreinigte Essigsäure in Gegenwart einer Brönstedt-Säure wie Schwefelsäure, Methansulfonsäure, Toluolsulfonsäure oder Phosphorsäure in einer Menge von 0,005 bis 0,05 mol/l verunreinigter Essigsäure und von 0,05 bis 1 Gewichtsprozent Wasser sowie von 3 bis 10 Äquivalenten, berechnet auf Aldehyd und Acetal, eines hochsiedenden Polyols mit dem allgemeinen Strukturglied 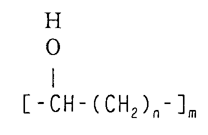 worin n für eine ganze Zahl von 0 bis 3 und m für eine ganze Zahl von größer als 3 steht, bei Temperaturen von 50 bis 118 °C über einen Zeitraum von 1 bis 5 Stunden reagieren läßt, und danach die reine Essigsäure durch fraktionierte Destillation aus dem Reaktionsprodukt gewinnt.

2. Verfahren nach Anspruch 1, dadurch gekennzeichnet, daß man als hochsiedendes Polyol Polyvinylalkohol, Rohrzucker, Dextrose, Stärke oder Zellulose einsetzt.

## Claims

1. A process for the removal of aldehydes and acetals from industrially prepared acetic acid, which comprises reacting the contaminated acetic acid in the presence of a Brönstedt acid such as sulfuric acid, methanesulfonic acid, toluenesulfonic acid or phosphoric acid in an amount of 0.005 to 0.05 mol/l of contaminated acetic acid and 0.05 to 1% by weight of water and of 3 to 10 equivalents, calculated on aldehyde and acetal, of a high-boiling polyhydric alcohol having the general structural member 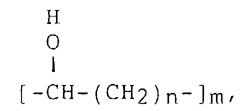 in which n is an integer from 0 to 3 and m is an integer greater than 3, at temperatures of 50 to 118°C for a period of 1 to 5 hours, and then isolating the pure acetic acid from the reaction product by fractional distillation.

2. The process as claimed in claim 1, wherein the high-boiling polyhydric alcohol used is a substance selected from the group consisting of poly(vinylalcohol), cane sugar, dextrose, starch or cellulose.

## Revendications

1. Procédé pour l'élimination d'aldéhydes et d'acétals à partir d'acide acétique préparé à l'échelle industrielle, caractérisé en ce que l'on laisse réagir l'acide acétique impur en présence d'un acide de Brönstedt comme l'acide sulfurique, l'acide méthanesulfonique, l'acide toluènesulfonique ou l'acide phosphorique, dans une quantité de 0,005 à 0,05 mol/l d'acide acétique impur et de 0,05 à 1% en poids d'eau ainsi que de 3 à 10 équivalents, rapportés à l'aldéhyde et à l'acétal, d'un polyol de point d'ébullition élevé avec la structure générale 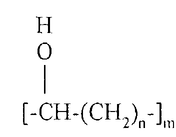 où n représente un nombre entier de 0 à 3 et m un nombre entier supérieur à 3, à des températures de 50 à 118°C sur une durée de 1 à 5 heures, et on récupère ensuite l'acide acétique pur par distillation fractionnée à partir du produit de la réaction.

2. Procédé selon la revendication 1, caractérisé en ce que l'on utilise comme polyol de point d'ébullition élevé du poly(alcool vinylique), du sucre brut, du dextrose, de l'amidon ou de la cellulose.
